# EUROPÄISCHE PATENTSCHRIFT

(11) **EP 0 504 660 B1**
(45) Veröffentlichungstag und Bekanntmachung des Hinweises auf die Patenterteilung: **19.08.1998**
(21) Anmeldenummer: 92103713.1
(22) Anmeldetag: 05.03.1992
(51) Int. Cl.: C07C 255/54, C09K 19/20, C07C 69/94, G02F 1/13, C09K 19/12

(54) **Flüssigkristalline Verbindungen**
Liquid crystal compounds
Composés cristaux liquides

(30) Priorität: 16.03.1991 DE 4108627
(43) Veröffentlichungstag der Anmeldung: 23.09.1992
(73) Patentinhaber: BASF Aktiengesellschaft, 67063 Ludwigshafen (DE)
(72) Erfinder: Bach, Volker, Dr., W-6730 Neustadt (DE); Etzbach, Karl-Heinz, Dr., W-6710 Frankenthal (DE); Siemensmeyer, Karl, Dr., W-6710 Frankenthal (DE); Wagenblast, Gerhard, Dr., W-6719 Weisenheim (DE)

(56) Entgegenhaltungen:
- WO-A-91/16295
- DE-A- 3 827 603
- GB-A- 2 188 048
- LIQUID CRYSTALS, Bd. 8, Nr. 6, 1990 Seiten 879-884 R. EIDENSCHINK ' Liquid-crystalline behaviour of molecules with tetrahedral symmetrie'

## Beschreibung

Die vorliegende Beschreibung betrifft flüssigkristalline Verbindungen der allgemeinen Formeln I und II

Z¹-(X¹-R¹-Y-M)ₘ I

Z²-(X²-R¹-Y-M)ₙ II.

Die Variablen haben folgende Bedeutung:
- Z¹: der Alkoholat- oder Säurerest Z¹/1 eines m-wertigen aliphatischen Alkohols bzw. einer m-wertigen aliphatischen Carbonsäure mit 3 bis 30 C-Atomen;
der Alkoholat- oder Säurerest Z¹/2 eines m-wertigen cycloaliphatischen Alkohols bzw. einer m-wertigen cycloaliphatischen Carbonsäure mit 5- oder 6-Ringgliedern;
für den Fall m = 3 der stickstoffhaltige Rest Z¹/3, 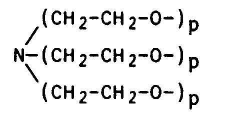 wobei p 1 oder 2 sein kann;
für den Fall m = 3 ein Rest der Strukturen Z¹/4a-d 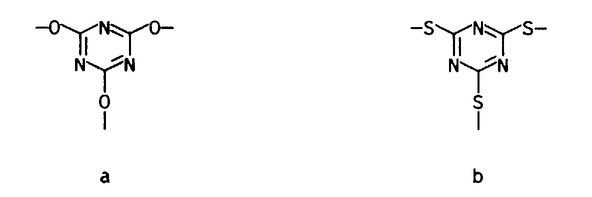 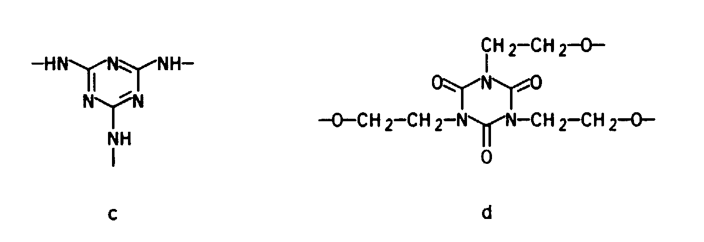 für den Fall m = 3 oder m = 4 der Säurerest Z¹/5 der Nitrilotriessigsäure bzw. der Ethylendiaminotetraessigsäure;
der Rest Z¹/6 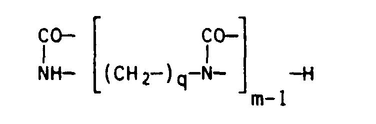 wobei q 2 oder 3 sein kann;
- Z²: der n-wertige Rest Z²/1 eines Benzols, 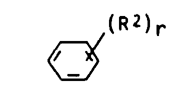 wobei R² Halogen, Cyano, Nitro, C₁- bis C₁₀-Alkyl, C₁- bis C₁₀-Alkoxy, C₁- bis C₁₀-Alkoxycarbonyl, C₁- bis C₁₀-Acyloxy oder Reste, die in vicinaler Stellung zu einem Ring verbunden sind, bedeuten kann; r für Null bis 3 steht und die Reste R² im Falle r > 1 gleich oder verschieden sein können;
der mehrkernige Rest Z²/2, 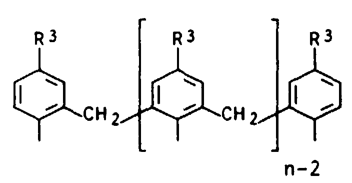 wobei R³ C₁- bis C₄-Alkyl, C₁- bis C₄-Alkoxy oder Halogen bedeuten kann;
für den Fall n = 3 der phosphorhaltige Rest Z²/3, 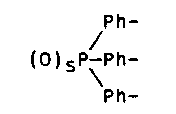 wobei s Null oder 1 sein kann;
- X¹: eine chemische Bindung oder eine -CO- Gruppe;
- X²: Sauerstoff, Schwefel, -CO-O-, -O-CO-, -SO₂-, -SO₂-O-, -O-SO₂-O-, -NR⁴-, -CO-NR⁴-, -NR⁴-O- oder -CO-N〈,
wobei R⁴ Wasserstoff oder C₁- bis C₈-Alkyl bedeuten kann, und die Maßgabe gilt, daß X² im Falle des mehrkernigen Restes Z²/2 nur -0- oder -O-CO- bedeutet;
- m: 3 bis 6, mit der Maßgabe, daß m gleich oder kleiner der Anzahl der C-Atome in Z¹ ist;
- n: 3 bis 6;
- R¹: eine C₂- bis C₂₀-Brücke mit 2 bis 12 Brückengliedern, die durch Sauerstoff, Schwefel oder -NR⁴⁻ unterbrochen sein können, und wobei diese Heteroeinheiten jeweils durch mindestens 2 C-Atome getrennt sind;
- Y: eine chemische Bindung, Sauerstoff, Schwefel, -CO-O-, -O-CO-, -NR⁴-, -CO-NR⁴- oder -NR⁴-CO-;
- M: eine mesogene Gruppe, die sich von einer Verbindung ableitet, welche in der flüssigkristallinen Phase eine Anisotropie der Dielektrizitätskonstanten ε mit |Δε| > 0,3 aufweist und/oder welche optisch aktiv ist und durch die allgemeinen Formeln III oder IV dargestellt wird:

-A-(B-A-)ₜ (D¹)ᵤ III

-A-(B-A-)ₜ (D²)ᵤ IV

in denen die Variablen die folgende Bedeutung haben:
- A: eine 1,4-Phenylen- oder 2,6-Naphthylengruppe, die bis zu zwei Stickstoffatome als Heteroatome enthalten kann, und die bis zu zwei der folgenden Substituenten tragen kann: Fluor, Chlor, Brom, Nitro oder Cyano;
eine 1,4-Cyclohexylengruppe, die bis zu zwei der folgenden Heteroatome in jeweils nicht benachbarter Stellung enthalten kann: Sauerstoff, Schwefel oder Stickstoff;
- B: eine chemische Bindung oder eines der folgenden Brückenglieder:
-CO-O-, -O-CO-, -CH₂-CH₂-, -CH₂-O-, -O-CH₂-, -CH₂-S-, -S-CH₂, -C≡C- , -CH=CH-, -CH=N-, -N=CH-, -N=N- , 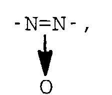 -CH=CH-CO-O- oder -O-CO-CH=CH-;
- D¹: einer der folgenden Substituenten:
Fluor, Chlor, Brom, Nitro, Cyano, -OCFH₂, -OCF₂H, -OCF₃, -O-CO-R⁵ oder -CO-O-R⁵,
wobei R⁵ ein linearer C₁- bis C₂₀-Alkylrest, der durch Sauerstoff unterbrochen sein kann, und der durch Fluor, Chlor, Brom, Cyano oder Methyl asymmetrisch substituiert sein kann, bedeutet;
- t: 1 bis 4, mit der Maßgabe, daß die Gruppen A und B verschieden sein können;
- u: 1, wenn D¹ bzw. D² mit einem aromatischen Ring verknüpft ist, und 1 oder 2, wenn D¹ bzw. D² mit einem Cyclohexylenring verknüpft ist,
- D²: eine der folgenden enantiomeren Gruppen:
eine lineare C₁- bis C₂₀-Alkylgruppe, die an ein oder zwei Kohlenstoffatomen durch Fluor, Chlor, Brom, Cyano, Trifluormethyl oder Methyl asymmetrisch substituiert ist, und die einmal durch die Gruppierung -CO-O- oder bis zu zweimal durch die Gruppierung -O- unterbrochen sein kann, oder
eine lineare C₃- bis C₆-Alkylgruppe, die durch die Gruppierung 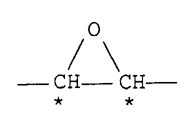 unterbrochen ist,
mit der Maßgabe, daß diese enantiomeren Gruppen D² auch durch -O-, -CO-O- oder -O-CO- mit A verknüpft sein können, ausgenommen die Verbindung Tetrakis(5-(4'-cyano-biphenylyl-4)-valeryloxymethyl)methan entsprechend einer Verbindung der Formel I.

Außerdem betrifft die Erfindung Verfahren zur Herstellung der Verbindungen I und II sowie deren Verwendung in Form fester oder flüssigkristalliner optisch anisotroper Medien für die Darstellung und Speicherung von Informationen.

Flüssigkristalline Verbindungen sind optisch anisotrope Stoffe, die im Gegensatz zu Flüssigkeiten eine Fernordnung der Moleküle aufweisen, wobei sie regelmäßig ein- oder zweidimensional angeordnet sein können und flüssigkristalline Mesophasen bilden. Im Hinblick auf die räumliche Anordnung der molekularen Bausteine innerhalb des Flüssigkristalls unterscheidet man im wesentlichen 3 Phasen:
die nematische, die cholesterische und die smektische Phase.

In nematischen Phasen sind die Einzelmoleküle eindimensional ausgerichtet. Ihr strukturelles Merkmal ist eine Parallelorientierung der Moleküllängsachsen bei statistischer Verteilung der Molekülschwerpunkte. Den Molekülen fehlt die laterale Kohäsion, so daß es nicht zu einem schichtenförmigen Aufbau wie bei den im folgenden beschriebenen smektischen Phasen kommt. Die Moleküle sind hinsichtlich ihrer langen Achsen zueinander verschiebbar, d.h. sie können frei aneinander vorbeigleiten, was ihre niedrige Viskosität bedingt. Daher sind nematische Phasen sehr viel dünnflüssiger als smektische Phasen.

Die Struktur der cholesterischen Phase, die nur mit optisch aktiven Molekülen realisierbar ist, ist eng verwandt mit der nematischen Phase. Sie wird daher auch häufig als chiral nematische Phase bezeichnet. Entsprechend der nematischen Phase sind die Moleküllängsachsen der flüssigkristallinen Verbindungen parallel zueinander angeordnet, jedoch ändert sich die Vorzugsrichtung der Moleküllängsachsen von Schicht zu Schicht regelmäßig um einen bestimmten Winkel. Innerhalb einer einzelnen Schicht existiert eine einheitliche Vorzugsrichtung, die gegenüber derjenigen innerhalb der benachbarten Schicht um einen konstanten Winkel gleichsinnig gedreht ist. So bildet sich über mehrere Schichten eine schraubenähnliche Anordnung der Moleküllängsachsen aus. Die Helixstruktur wird durch den chiralen Aufbau der beteiligten Moleküle hervorgerufen.

Den smektischen Phasen liegt ein zweidimensionaler Aufbau zugrunde. Durch intermolekulare Wechselwirkungen werden die langgestreckten, stäbchen-förmigen, parallel ausgerichteten Moleküle zu Schichten zusammengefügt und diese mit jeweils gleichen Abständen gestapelt. Hierbei können verschiedene Modifikationen auftreten, die sich in der Anordnung der Moleküle innerhalb der Schichten unterscheiden. Bekannt sind die smektischen Phasen S_{A} bis S_{I}. Beispielsweise können die Schwerpunkte der Moleküle innerhalb einer Schicht statistisch (S_{A}- und S_{C}-Phase) oder regelmäßig (S_{B}-Phase) angeordnet sein. Die Moleküllängsachsen können parallel oder geneigt zur Senkrechten auf der Schichtebene liegen. Die Moleküle können die Schichtebene nicht verlassen, da zwischen den Molekülenden kaum Wechselwirkungskräfte bestehen. Obwohl sich dadurch die Schichten noch leicht gegeneinander verschieben lassen, ist aufgrund des höheren Ordnungszustandes (zweidimensionaler Aufbau) die Viskosität smektischer Phasen höher als die nematischer Phasen.

Ferner sind smektisch flüssigkristalline Phasen bekannt, die in Abwesenheit eines äußeren elektrischen Feldes eine elektrische Spontanpolarisation aufweisen. Durch Anlegen eines äußeren elektrischen Feldes kann diese Polarisation umorientiert werden, weswegen diese Phasen als ferroelektrisch smektisch flüssigkristalline Phasen bezeichnet werden. Ein typisches Beispiel ist die chirale S_{C}-Phase (S_{C}*-Phase). Aufgrund ihrer anisotropen optischen und dielektrischen Eigenschaften lassen sie sich für elektrooptische Anzeigeelemente - kürzer Displays genannt - nutzen. So ist mit ferroelektrischen Displays auf der Basis von S_{C}*-Phasen ein extrem schnelles Einschreiben und Löschen von Zeichen möglich.

Die folgende Symbolik dient zur Kennzeichnung der flüssigkristallinen Phasen oder des flüssigkristallinen Verhaltens:
- S: symbolisiert eine smektisch flüssigkristalline Phase oder das smektisch flüssigkristalline Verhalten;
- N: symbolisiert eine nematisch flüssigkristalline Phase oder das nematisch flüssigkristalline Verhalten;
- N*: symbolisiert eine cholesterisch (chiral nematisch) flüssigkristalline Phase oder das cholesterisch (chiral nematisch) flüssigkristalline Verhalten, wobei
- *: bedeutet, daß die flüssigkristalline Verbindung ein chirales, d.h. optisch aktives Zentrum enthält und sich daher auch optisch aktive flüssigkristalline Phasen bilden können.

Beim Schmelzen einer festen flüssigkristallinen Verbindung entsteht zunächst als flüssigkristalline Phase beispielsweise eine smektische Phase, die bei weiterer Temperaturerhöhung bei der für jede Verbindung charakteristischen Phasenübergangstemperatur entweder in eine weitere, beispielsweise nematisch flüssigkristalline Phase oder am Klärpunkt in die optisch isotrope Schmelze übergeht. Beim Abkühlen der Schmelze bilden sich bei den entsprechenden Umwandlungstemperaturen die flüssigkristallinen Phasen und schließlich der kristalline Flüssigkristall wieder aus. Diese wechselseitige Umwandlung der flüssigkristallinen Verbindung bezeichnet man als enantiotrope Umwandlung.

Unter den Flüssigkristallen existieren Verbindungen, bei denen es möglich ist, die einmal erzeugte flüssigkristalline Phase beim Abkühlen einzufrieren. Dazu wird die flüssigkristalline Schmelze unter eine bestimmte Temperatur abgekühlt, so daß ein optisch anisotroper Festkörper resultiert, der nicht kristallin, sondern glasartig ist.

Flüssigkristalline Verbindungen, die glasartig erstarrende Eigenschaften zeigen, finden sich sowohl im Bereich der niedermolekularen organischen als auch im Bereich der polymeren organischen Verbindungen. Bei den bekannten polymeren Flüssigkristallen dienen u.a. Polyacryl- und Polymethacrylketten als polymere Hauptketten. Über polymeranaloge Umsetzungen werden Strukturelemente von niedermolekularen flüssigkristallinen Verbindungen als Seitengruppen an das Rückgrat dieser Polymeren geknüpft.

Aus der EP-B 90 282 sind flüssigkristalline Phasen bildende Polymere auf der Basis von Acrylsäure- und Methacrylsäureestern und -amiden bekannt. Die über die Ester- und Amidfunktion an die Polymerhauptkette abgeknüpften Seitenketten, bestehend aus einem flexiblen, abstandhaltenden, langkettigen Molekülteil - auch Spacer genannt - und einer mesogenen Gruppe oder eines pleochroitischen Farbstoffs, nehmen Einfluß auf die Bildung von flüssigkristallinen Phasen.

Als Spacer kommen Alkylengruppen mit 2 bis 12 C-Atomen in Betracht, die linear oder verzweigt und durch Sauerstoff, Schwefel und/oder R⁶-N〈-Gruppen unterbrochen sein können, wobei R⁶ Wasserstoff oder gegebenenfalls ein substituiertes Alkyl ist.

Als mesogene Gruppen sind beispielsweise die in Kelker und Hatz, Handbook of Liquid Crystals, Verlag Chemie 1980, Seite 87 bis 113, genannten Gruppen möglich.

In Abhängigkeit von den Spacern und/oder den mesogenen Gruppen können die Polymeren flüssigkristalline Phasen bilden und als solche sowie mit niedermolekularen Flüssigkristallen in elektrooptischen Anzeigen eingesetzt werden.

Prinzipiell sind die elektrooptischen Anzeigen aus einer 6 bis 30 µm dicken flüssigkristallinen Schicht, welche sich zwischen zwei Glasplatten befindet, von denen jede auf ihrer Innenseite mit einer Elektrodenschicht bedeckt ist, und mindestens eine transparent sein muß, aufgebaut. Als transparente, elektrisch leitende Deckschichten werden vor allem mit Antimon dotierte Zinnoxidschichten und mit Zinnoxid dotierte Indiumoxidschichten verwendet.

Aus der DE-A 39 17 196 gehen Monomere mit der allgemeinen Formel V hervor, 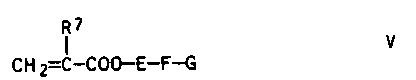 worin der Rest R⁷ ein Wasserstoff- oder Chloratom oder eine Methylgruppe, die Variable E einen flexiblen, abstandhaltenden Molekülteil, die Variable F einen aus mindestens drei linear oder angenähert linear miteinander verknüpften aromatischen Kernen aufgebauten mesogenen Molekülteil, welcher zumindest eine 2,6-Naphthylengruppe enthält, und die Variable G einen optisch aktiven chiralen Molekülteil bedeuten. Diese Monomeren finden Verwendung zur Herstellung von Polymerisaten mit chiralen mesogenen Seitengruppen, welche ferroelektrisch smektisch flüssigkristallines Verhalten zeigen.

Nachteilig bei der Verwendung von polymeren Flüssigkristallen sind die nur langsam ablaufenden Schaltvorgänge. Ein weiterer Nachteil ist, daß die polymeren Flüssigkristalle nicht wie die niedermolekularen Verbindungen mit einem einheitlichen, definierten Molekulargewicht synthetisierbar sind.

Im Bereich der niedermolekularen Flüssigkristalle sind flüssigkristalline Verbindungen mit Glasphasen bekannt, die aufgrund ihrer charakteristischen Phaseneigenschaften in Technik und Elektrooptik anwendbar sind.

In der DE-A 37 03 640 werden glasartige flüssigkristalline 2-substituierte 1,4-Bis-(4-substituierte-benzoyloxy)-benzole beschrieben. Sie werden allein, in Gemischen untereinander oder mit anderen flüssigkristallinen oder nicht flüssigkristallinen Stoffen als anisotrope feste optische Medien zur Herstellung optischer Bauteile sowie für thermoelektrooptische Speicherdisplays bei Zimmertemperatur eingesetzt.

Entsprechende Anwendungsbereiche existieren für die aus der DE-A 38 30 968 bekannten 1,4-Bis-(2,3,4-substituierten benzoyloxy)-benzole, die glasartig erstarrende nematische Phasen bilden.

Nach der Lehre von DE-A 38 27 603 sind chirale, smektische flüssigkristalline Verbindungen der allgemeinen Formel VI bekannt,

M₁*-K-M₂* VI

wobei M₁* und M₂* gleiche chirale, smektogene Gruppen sind und das Brückenglied -K- für eine bivalente Gruppe steht. Sie zeigen die Eigenschaft, daß sie gleichzeitig beim Abkühlungsprozeß aus der kristallinen Phase glasartig erstarren.

Die bislang bekannten niedermolekularen flüssigkristallinen Verbindungen mit Glasphasen haben die gemeinsame Eigenschaft, daß ihre Glasphasen keine lang andauernde Stabilität aufweisen und daher bei Raumtemperatur nach kurzer Zeit einen kristallinen Ordnungszustand einnehmen. Die zuvor eingeschriebene Information geht durch diesen Kristallisationsprozeß verloren. Nachteilig ist ferner ihre niedrige Anisotropie der Dielektrizitätskonstanten Δε, die für die Ausrichtung der Moleküle im elektrischen Feld maßgebend ist. Die dielektrische Anisotropie ist die Differenz zwischen den parallel und senkrecht zur resultierenden Vorzugsrichtung, die sich aus der Ausrichtung der gesamten Moleküllängsachsen ergibt, gemessenen Dielektrizitätskonstanten ε. Die parallel zur Vorzugsrichtung gemessenen Werte für die Dielektrizitätskonstante ε werden mit ε_{∥} und die senkrecht dazu gemessenen Werte werden mit ε_{⊥} bezeichnet. Die DK-Anisotropie (Δε = ε_{∥} - ε_{⊥}) kann sowohl positiv als auch negativ sein. Ist ε_{∥} größer als ε_{⊥} und somit Δε positiv, dann stellt sich der Flüssigkristall so ein, daß seine optische Achse parallel zum Feld liegt. Ist Δε negativ, stellt sich die optische Achse senkrecht zum Feld ein.

Der vorliegenden Erfindung lag daher die Aufgabe zugrunde, flüssigkristalline Verbindungen zu finden, die eine hohe dielektrische Anisotropie aufweisen, einheitliche Substanzen mit einem definierten Molekulargewicht, niedrigviskos, in reiner Form reproduzierbar und bei den in der Praxis vorkommenden tiefen Temperaturen schnell schaltbar sind sowie die durch die Schaltvorgänge geschriebenen Informationen speichern können.

Demgemäß wurden die eingangs definierten flüssigkristallinen Verbindungen I und II gefunden.

Ferner wurden verschiedene, im folgenden näher beschriebene Verfahren für die Herstellung der flüssigkristallinen Verbindungen I und II gefunden sowie deren Verwendung allein, in Mischungen untereinander und mit anderen flüssigkristallinen und/oder nicht flüssigkristallinen Verbindungen in Form fester oder flüssigkristalliner optisch anisotroper Medien für die Darstellung und Speicherung von Informationen.

Den durch die allgemeinen Formeln I und II

Z¹-(X¹-R¹-Y-M)ₘ I

Z²-(X²-R¹-Y-M)ₙ II

beschriebenen flüssigkristallinen Verbindungen liegen zentrale Molekülteile - auch Zentraleinheiten genannt - zugrunde, die durch die Variablen Z¹ und Z² symbolisiert werden. Die Variable X¹ bezeichnet eine chemische Bindung oder eine -CO-Gruppe, die Variable X² steht ganz allgemein für Heteroeinheiten, die wie X¹ an eine C₂- bis C₂₀-Brücke gebunden ist, welche durch die Variable R¹ gekennzeichnet und als Spacer bezeichnet wird. Die Variable Y steht wiederum für eine chemische Bindung oder für Heteroeinheiten und die Variable M für mesogene Gruppen, die sich von Verbindungen ableiten, welche in der flüssigkristallinen Phase eine Anisotropie der Dielektrizitätskonstanten ε mit |Δε| > 0,3 aufweisen und/oder welche optisch aktiv sind.

Die Variable m bedeutet eine Zahl von 3 bis 6, mit der Maßgabe, daß m gleich oder kleiner der Anzahl der C-Atome in Z¹ ist, die Variable n bedeutet eine Zahl von 3 bis 6.

Die Zentraleinheit Z¹ steht für Reste, die sich beispielsweise von folgenden Verbindungen ableiten:
von aliphatischen Alkoholen wie
   Glycerin,
   1,2,4-Butantriol,
   2-Methyl-2-hydroxymethyl-1,3-propandiol,
   2-Ethyl-2-hydroxymethyl-1,3-propandiol,
   1,2,3,4-Butantetraol,
   Pentaerythrit,
   Xylit,
   Mannit und
   Sorbit,
von aliphatischen Carbonsäuren wie
   1,2,3-Propantricarbonsäure,
   1,1,4-Butantricarbonsäure,
   1,2,3,4-Butantetracarbonsäure,
   Zitronensäure und
   2-Hydroxy-nonadecyl-1,2,3-tricarbonsäure,
von cycloaliphatischen Alkoholen mit 5- oder 6-Ringgliedern wie
   1,2,3,4-Tetrahydroxy-cyclopentan,
   1,2,3-Trihydroxy-cyclohexan,
   1,2,4-Trihydroxy-cyclohexan,
   1,3,5-Trihydroxy-cyclohexan,
   1,2,3,4-Tetrahydroxy-cyclohexan,
   1,2,3,5-Tetrahydroxy-cyclohexan,
   1,2,4,5-Tetrahydroxy-cyclohexan,
   1,2,3,4,5-Pentahydroxy-cyclohexan und
   1,2,3,4,5,6-Hexahydroxy-cyclohexan,
von cycloaliphatischen Carbonsäuren mit 5- oder 6-Ringgliedern wie
   1,2,3-Cyclopentantricarbonsäure,
   1,2,4-Cyclopentantricarbonsäure,
   2-Methyl-1,2,3-cyclopentantricarbonsäure,
   3-Methyl-1,2,4-cyclopentantricarbonsäure,
   1,1,2,2-Cyclopentantetracarbonsäure,
   1,2,2,4-Cyclopentantetracarbonsäure,
   1,1,3,3-Cyclopentantetracarbonsäure,
   1,2,3,4-Cyclopentantetracarbonsäure,
   1,2,3,4,5-Cyclopentanpentacarbonsäure,
   1,1,4-Cyclohexantricarbonsäure,
   1,2,4-Cyclohexantricarbonsäure,
   1,3,5-Cyclohexantricarbonsäure,
   1,1,3,3-Cyclohexantetracarbonsäure,
   1,1,4,4-Cyclohexantetracarbonsäure,
   1,2,3,4-Cyclohexantetracarbonsäure,
   1,2,4,5-Cyclohexantetracarbonsäure,
   1,1,3,3,5-Cyclohexanpentacarbonsäure und
   1,2,3,4,5,6-Cyclohexanhexacarbonsäure,
von Alkoholaminen wie
   Triethanolamin,
   Triisopropanolamin und
   Aminoethylethanolamin,
von Triazinderivaten wie
   Cyanursäure,
   Thiocyanursäure,
   Melamin und
   Trishydroxyethylisocyanurat,
von Nitrilotriessigsäure und Ethylendiaminotetraessigsäure,
und von dem Rest, 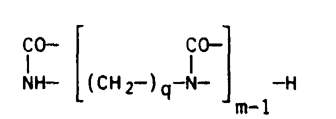 wobei q 2 oder 3 sein kann.

Besonders gut geeignete Zentraleinheiten Z¹ sind Glycerin, Pentaerythrit, Mannit und Zitronensäure.

Die Zentraleinheit Z² steht für n-wertige Reste Z²/1 eines Benzols, 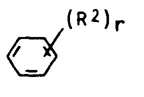 wobei R² Halogen, Cyano, Nitro, C₁- bis C₁₀-Alkyl, C₁- bis C₁₀-Alkoxy, C₁- bis C₁₀-Alkoxycarbonyl, C₁- bis C₁₀-Acyloxy oder Reste, die in vicinaler Stellung zu einem Ring verbunden sind, bedeuten kann, r für Null bis 3 steht und die Reste R² im Falle r > 1 gleich oder verschieden sein können.

Beispielsweise steht die Zentraleinheit Z² für Reste Z²/1, die sich von folgenden Verbindungen wie
1,2,3-Trihydroxybenzol,
1,2,4-Trihydroxybenzol,
1,3,5-Trihydroxybenzol,
1,2,3,4-Tetrahydroxybenzol,
1,2,3,5-Tetrahydroxybenzol,
1,2,4,5-Tetrahydroxybenzol,
Hexahydroxybenzol,
1,2,3-Benzoltricarbonsäure,
1,2,4-Benzoltricarbonsäure,
1,3,5-Benzoltricarbonsäure,
3,4,5-Trihydroxybenzoesäure,
3,4,5-Trihydroxybenzoesäuremethylester,
1,2,3-Trihydroxytoluol,
2,4,5-Trihydroxytoluol,
2,4,6-Trihydroxytoluol,
3,4,5-Trihydroxytoluol,
Pyromellitsäureanhydrid,
1,2,3,4-Tetrahydroxynaphthalin,
2,3,4-Trihydroxyanthracen,
1,2,3-Trihydroxy-9,10-anthrachinon und
1,2,4-Trihydroxy-9,10-anthrachinon
ableiten.

Ferner steht die Variable Z² für den mehrkernigen Rest Z²/2 mit der Formel, 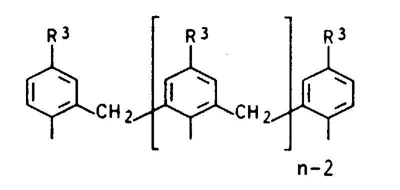 wobei R³ C₁- bis C₄-Alkyl, C₁- bis C₄-Alkoxy oder Halogen bedeuten kann.

Für den Fall n = 3 kann die Zentraleinheit Z²/3 für den phosphorhaltigen Rest stehen, 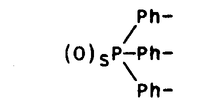 wobei S Null oder 1 sein kann.

Bevorzugte Verbindungen sind
1,3,5-Trihydroxybenzol,
Pyromellitsäureanhydrid und
3,4,5-Trihydroxybenzoesäuremethylester sowie
der Rest

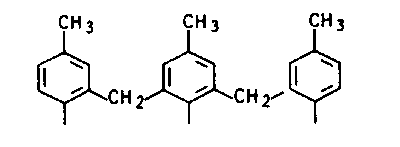

Bei den Variablen X² handelt es sich im wesentlichen um die Heteroeinheiten
Sauerstoff, Schwefel, -CO-O-, -O-CO-, -SO₂-, -SO₂-O-, -O-SO₂-O-, -NR⁴-, -CO-NR⁴-, -NR⁴-O- oder -CO-N〈,
wobei R⁴ Wasserstoff oder C₁- bis C₈-Alkyl bedeuten kann, und die Maßgabe gilt, daß X² im Falle des mehrkernigen Restes Z²/2 nur -O- oder -O-CO-bedeutet.

Bevorzugt sind Sauerstoff, -CO-O- und -O-CO-.

Die Variable R¹ steht für eine C₂- bis C₂₀-Brücke mit 2 bis 12 Brückengliedern, die durch Sauerstoff, Schwefel oder -NR⁴- unterbrochen sein können, und wobei diese Heteroeinheiten jeweils durch mindestens 2 C-Atome getrennt sind.

Gut geeignete Brückenglieder R¹ sind beispielsweise
-(CH₂)₂-, -(CH₂)₃-, -(CH₂)₄-, -(CH₂)₅-, -(CH₂)₆-, -(CH₂)₇-, -(CH₂)₈-, -(CH₂)₁₀-, -(CH₂)₁₂-, -(CH₂-O-CH₂)₂-, -(CH₂)₂-S-(CH₂)₂-,

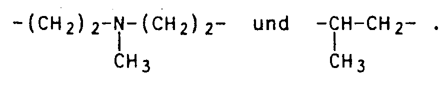

Besonders gut geeignete Brückenglieder R¹ sind -(CH₂)₅-, -(CH₂)₆-, -(CH₂)₇- und -(CH₂)₁₀-.

Die Variable Y kennzeichnet eine chemische Bindung, Sauerstoff, Schwefel, -CO-O-, -O-CO-, -NR⁴-, -CO-NR⁴- oder -NR⁴-CO-, wobei R⁴ Wasserstoff oder C₁- bis C₈-Alkyl bedeuten kann.

Besonders bevorzugt ist Sauerstoff.

Bevorzugte mesogene Gruppen M sind Gruppen der allgemeinen Formel III,

-A-(B-A-)ₜ (D¹)ᵤ III

in der die Variable A
   eine 1,4-Phenylen- oder 2,6-Naphthylengruppe, die bis zu zwei Stickstoffatome als Heteroatome enthalten kann, und die bis zu zwei der folgenden Substituenten Fluor, Chlor, Brom, Nitro oder Cyano tragen kann, oder eine 1,4-Cyclohexylengruppe, die bis zu zwei der folgenden Heteroatome Sauerstoff, Schwefel oder Stickstoff in jeweils nicht benachbarter Stellung enthalten kann,
die Variable B
   eine chemische Bindung oder eine der folgenden Brückenglieder -CO-O-, -O-CO-, -CH₂-CH₂-, -CH₂-O-, -O-CH₂-, -CH₂-S-, -S-CH₂-, -C≡C- -CH=CH-, -CH=N-, -N=CH-, -N=N-, 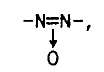 -CH=CH-CO-O- oder -O-CO-CH=CH-,
die Variable D¹
   eine der folgenden Substituenten Fluor, Chlor, Brom, Nitro, Cyano, -OCFH₂, -OCF₂H, -OCF₃, -O-CO-R⁵ oder -CO-O-R⁵, wobei R⁵ ein linearer C₁- bis C₂₀-Alkylrest, der durch Sauerstoff unterbrochen sein kann, und der durch Fluor, Chlor, Brom, Cyano oder Methyl asymmetrisch substituiert sein kann, bedeutet,
die Variable t
   eine Zahl von 1 bis 4, mit der Maßgabe, daß die Gruppen A und B verschieden sein können, und
die Variable u
   1, wenn D¹ mit einem aromatischen Ring verknüpft, und
   1 oder 2, wenn D¹ mit einem Cyclohexylenring verknüpft ist,
bedeuten.

Eine besonders bevorzugte mesogene Gruppe M ist

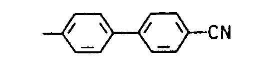

Weitere bevorzugte mesogene Gruppen M sind Gruppen der allgemeinen Formel IV

-A-(B-A-)ₜ (D²)ᵤ IV

in der D² eine der folgenden enantiomeren Gruppen ist:
eine lineare C₁- bis C₂₀-Alkylgruppe, die an ein oder zwei Kohlenstoffatomen durch Fluor, Chlor, Brom, Cyano, Trifluormethyl oder Methyl asymmetrisch substituiert ist, und die einmal durch die Gruppierung -CO-O- oder bis zu zweimal durch die Gruppierung -O- unterbrochen sein kann,
oder
eine lineare C₃- bis C₆-Alkylgruppe, die durch die Gruppierung 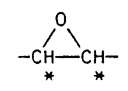 unterbrochen ist,
mit der Maßgabe, daß diese enantiomeren Gruppen D² durch -O-, -CO-O- oder -O-CO- mit A verknüpft sein können.

Beispiele gut geeigneter enantiomerer Gruppen D² sind: 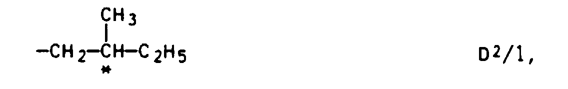 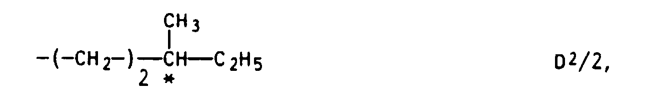 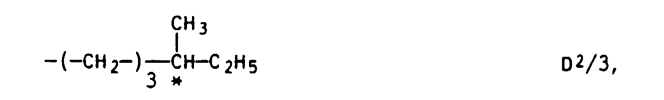 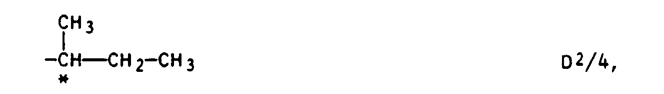 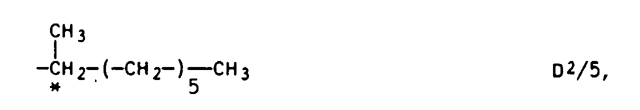 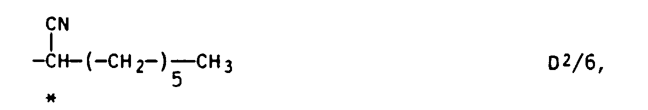 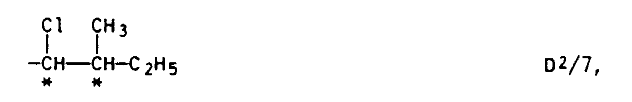 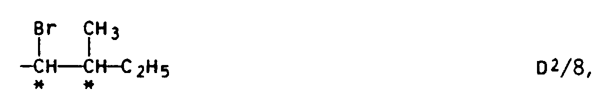 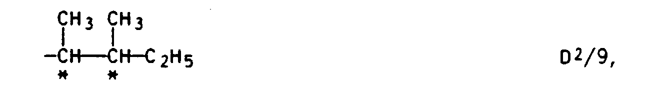 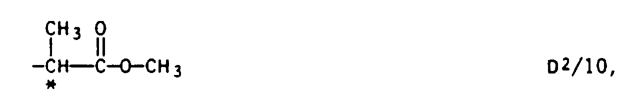 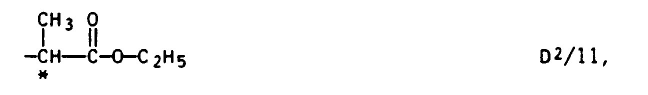 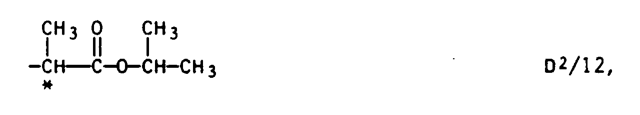 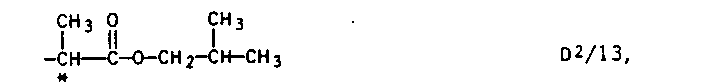 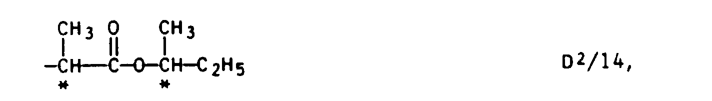 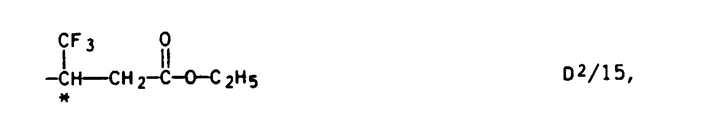 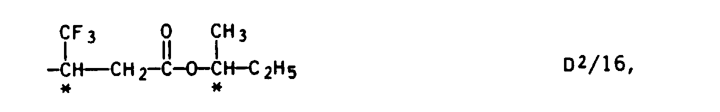 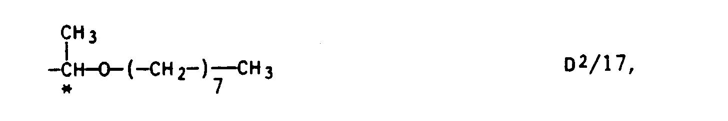 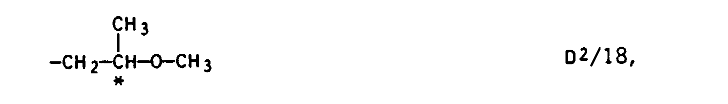 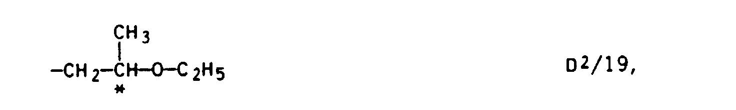 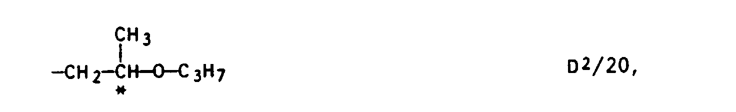 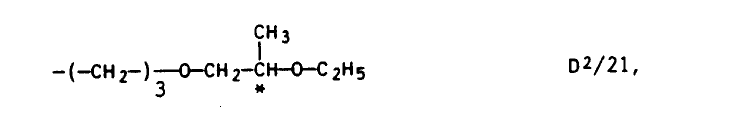 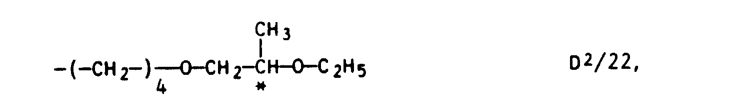 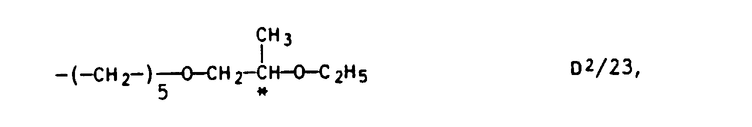 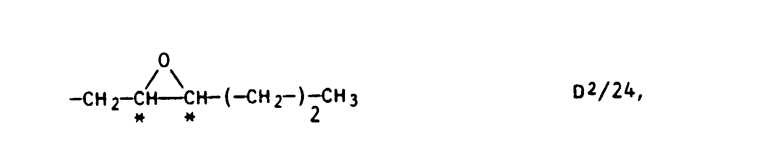 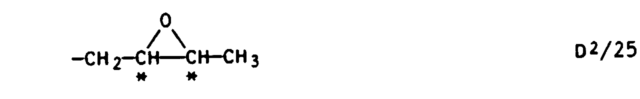 und 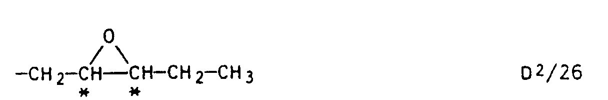 von denen die Gruppen D²/6, D²/7, D²/11 D²/15, D²/17 bis D²/21 und D²/24 besonders gut geeignet sind.

Eine besonders bevorzugte mesogene Gruppe ist

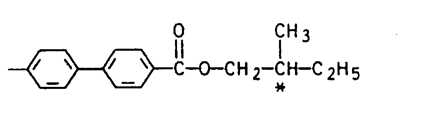

Für die Herstellung der flüssigkristallinen Verbindungen I gilt allgemein das Prinzip, Alkohole Z¹(H)ₘ mit Carbonsäurehalogeniden M-Y-R¹-CO-Hal, wobei Hal für Chlor oder Brom steht, oder Carbonsäuren Z(H)ₘ oder deren Derivate wie Säurechloride Z¹(Cl)ₘ oder Anhydride (Z¹)₂O mit Alkoholen M-Y-R¹-OH umzusetzen.

Im einzelnen sind die flüssigkristallinen Verbindungen I nach folgenden Methoden erhältlich:

Man geht von einer Carbonsäure M-Y-R¹-COOH aus und setzt diese in bekannter Weise mit Thionylchlorid in Gegenwart von Dimethylformamid zum Carbonsäurechlorid M-Y-R¹-CO-Cl um. Im nachfolgenden Schritt ist durch Umsetzung des Säurechlorids M-Y-R¹-CO-Cl mit einem Alkohol Z¹(H)ₘ in Gegenwart von Pyridin die Verbindung I erhältlich.

Die Alkoholyse von Carbonsäurechloriden ist in zahlreichen Ausgestaltungen allgemein bekannt, so daß sich nähere Ausführungen hierzu erübrigen. Es ist lediglich hinzuzufügen, daß es sich meistens empfiehlt, das Säurechlorid im Überschuß einzusetzen, um den Alkohol quantitativ umzusetzen. Nach der Isolierung des Produktes I kann eine zusätzliche Reinigung mittels Gelchromatographie und anschließende Umkristallisation vorgenommen werden, wozu sich meistens Methylenchlorid-Methanol-Gemische eignen.

Eine weitere Möglichkeit zur Herstellung der flüssigkristallinen Verbindung I besteht in der sauer katalysierten Veresterung der Carbonsäuren Z¹(H)ₘ, der Säurechloride Z¹(Cl)ₘ und der Anhydride (Z¹)₂O mit Alkoholen M-Y-R¹-OH.

Die Veresterung ist in zahlreichen Ausgestaltungen hinsichtlich der Säure, der Temperatur und der Reaktionszeit allgemein bekannt.

Eine Variante zur Umsetzung von Z¹(H)ₘ mit Carbonsäurechloriden stellt die Umsetzung von Z¹(H)ₘ mit ω-Bromcarbonsäurechloriden Br-R¹-CO-Cl und die anschließende Veretherung des Zwischenproduktes Z¹-CO-R¹-Br mit einer alkoholischen Verbindung HO-M zur Verbindung I dar.

Für die Herstellung der flüssigkristallinen Verbindungen II gilt allgemein das Prinzip, phenolische Verbindungen Z²(OH)ₙ mit Verbindungen, die sich von Verbindungen des Typs M-Y-R¹-X²-H ableiten oder deren Derivate sind, oder mit einem Alkylbromid M-Y-R¹-Br umzusetzen.

Ferner erhält man die Verbindungen II in an sich bekannter Weise durch Umsetzung der Verbindung Z²(OH)ₙ mit ω-Bromcarbonsäurechlorid Br-R¹-CO-Cl über die Stufe eines Zwischenproduktes Z²(-O-CO-R¹-Br)ₙ, welches anschließend mit der Verbindung HO-M verethert wird.

Eine weitere Möglichkeit zur Herstellung der Verbindungen II stellt die Veresterung von Carbonsäuren Z²(COOH)ₙ, Säurechloriden Z²(COCl)ₙ und Anhydriden (Z²CO)₂ mit Verbindungen des Typs M-Y-R¹-OH dar, die auf übliche Weise durchgeführt wird.

Die erfindungsgemäßen flüssigkristallinen Verbindungen I und II sind in hoher Reinheit mit einem einheitlichen Molekulargewicht herstellbar, schnell schaltbar und besitzen eine hohe dielektrische Anisotropie. Außerdem neigen sie kaum zur Kristallisation.

Die erfindungsgemäßen flüssigkristallinen Verbindungen I und II eignen sich hervorragend allein, in Mischungen untereinander und mit anderen flüssigkristallinen und/oder nicht flüssigkristallinen Verbindungen in Form fester oder flüssigkristalliner optisch anisotroper Medien für die Darstellung und Speicherung von Informationen. Darüber hinaus eignen sie sich zur Herstellung anisotroper fester optischer Medien für optische Bauteile wie Kompensatoren, Polarisatoren, Prismen, Platten mit optischer Drehung und elektrooptischen Speicherdisplays.

### Beispiele

Die Phasenübergänge wurden im Polarisationsmikroskop (Leitz, Ortholux II Pol BK, Mettler-Heiztisch FP 82 HT, Mettler-Steuergerät FP 80), Heizrate 10°C/min, und durch DSC-Messungen (Perkin-Elmer, DSC-7), Heizrate 20°C/min, bestimmt.

Die folgende Symbolik kennzeichnet die Phasen. Ein Phasenwechsel findet bei den gemessenen Phasenumwandlungstemperaturen statt.
k = kristallin
g = Glasstufe
n = nematisch
ch = cholesterisch
s = smektisch
i = isotrop

Die Umwandlungstemperaturen (°C) der erfindungsgemäßen Verbindungen sind jeweils bei den Beispielen angegeben. Die Temperaturen in Klammern bedeuten monotrope Umwandlungen der Phasen. Darunter sind Phasenübergänge zu verstehen, die nur in einer Richtung ablaufen, z.B. dann, wenn der flüssigkristalline Zustand nur durch Unterkühlung der Schmelze erreichbar ist. Der Schmelzpunkt liegt dann höher als der Klärpunkt.

### Herstellung der flüssigkristallinen Verbindungen I

### Beispiel 1

### Pentaerythrit-tetra-(ω-(4'-cyanbiphenyl-4-oxy)-heptylcarboxylat)

a) 8-(4'-Cyanbiphenyl-4-oxy)-octansäure
   Ein Gemisch, bestehend aus 41,3 g (212 mmol) 4-(4'-Hydroxybiphenyl)-carbonitril, 56,7 g (254 mmol) 8-Bromoctansäure, 42,1 g (254 mmol) Kaliumjodid, 70,1 g (508 mmol) Kaliumcarbonat und 400 ml 2-Butanon, wurde 32 Stunden unter Rückfluß erhitzt. Der nach dem Abkühlen angefallene Rückstand wurde abgetrennt, mit 2-Butanon gewaschen, in verd. Salzsäure aufgenommen, einige Minuten gerührt, isoliert und mit Wasser gewaschen. Schließlich wurde das Produkt zweimal aus Toluol umkristallisiert.
   Ausbeute: 30 %
   Schmp.: 147-148°C
b) 8-(4'-Cyanbiphenyl-4-oxy)-octansäurechlorid
   Eine Lösung, bestehend aus 17,8 g (52,7 mmol) 8-(4'-Cyanbiphenyl-4-oxy)-octansäure, 7,5 g (63,3 mmol) Thionylchlorid und 3 Tropfen Dimethylformamid in 160 ml Toluol, wurde 1,5 Stunden zum Sieden erhitzt. Nach Entfernung der leicht flüchtigen Bestandteile im Vakuum bei 80°C wurde der Rückstand bei Raumtemperatur im Vakuum getocknet.
   Ausbeute: 95 %
   Schmp.: 57-58°C
c) Pentaerythrit-tetra-(ω-(4'-cyanbiphenyl-4-oxy)-heptylcarboxylat)
   Zu einer Lösung, bestehend aus 0,9 g (6,7 mmol) Pentaerythrit, 3,2 g (40 mmol) Pyridin und 90 ml Methylenchlorid, wurden 11,8 g (33,3 mmol) 8-(4'-Cyanbiphenyl-4-oxy)-octansäurechlorid zugegeben, die dann unter Feuchtigkeitsausschluß bei Raumtemperatur über Nacht gerührt wurde. Nach der Aufarbeitung wurde das Rohprodukt durch Chromatographie über Kieselgel (Elutionsmittel:Toluol/Essigester=3/1) und Umkristallisation aus Methylenchlorid/Methanol gereinigt.
   Ausbeute: 38 %
   ¹H-NMR (CDCl₃, TMS):
   - δ (ppm):: 1,1-1,93 (m,40H,CH₂); 2,3 (t,8H,CH₂); 4,0 (t,8H,CH₂); 4,12 (s,8H,CH₂); 7,0 (d,8H,Arom.-H); 7,53 (d,8H,Arom.-H); 7,6-7,8 (m,16H,Arom.-H)
   - Phasenübergänge:: g - s 20°C
   s - n 80°C
   n - i 95°C

### Beispiel 2

### Mannit-hexa-(ω-(4'-cyanbiphenyl-4-oxy)-pentylcarboxylat)

Die Herstellung erfolgte gemäß Beispiel 1a), 1b) und 1c). Anstelle von Pentaerythrit wurde Mannit eingesetzt.
Ausbeute: 35 %
¹H-NMR (CDCl₃, TMS):
- δ (ppm):: 1,38-1,9 (m,36H,CH₂); 2,27-2,52 (m,12H,CH₂);
3,88-4,1 (m, 14H, CH₂) ; 4,2-4,38 (m,2H,CH₂) ; 5, 0-5, 2 (m,2H,CH); 5,43-5,55 (m,2H,CH); 6,9-7,07 (m,12H,Arom.-H);
7,4-7,75 (m,36H,Arom.-H)
- Phasenübergänge:: g - ch 34°C
ch - i 115°C

### Beispiel 3

### Mannit-hexa-(ω-(4'-cyanbiphenyl-4-oxy)-heptylcarboxylat)

Die Herstellung erfolgte gemäß Beispiel 1a), 1b) und 1c). Anstelle von Pentaerythrit wurde Mannit eingesetzt.
Ausbeute: 36 %
¹H-NMR (DMSO-d₆, TMS):
- δ (ppm):: 1,0-1,8 (m,60H,CH₂) ; 2,1-2,4 (m,12H,CH₂); 3,7-4,1 (m,14H,CH₂); 4,2-4,32 (m,2H,CH₂); 4,9-5,1 (m,2H,CH); 5,35-5,5 (m,2H,CH); 6,8-7,1 (m,12H,Arom.-H); 7,5-8,0 (m, 36H, Arom.-H)
- Phasenübergänge:: g - s 27°C
s - ch 116°C
ch - i 117°C

### Beispiel 4

### Bis-1,2,3-propantricarbonsäuretri-(4-(4'-undecyloxy)biphenylcarbonsäure-(S-2-methyl-)butylester)-yl-adipat

a) 4-(4'-ω-Hydroxyundecyloxy)-biphenylcarbonsäure(S-2-methyl-)butylester
   36,1 g (127 mmol) 4-(4'-Hydroxybiphenyl)-carbonsäure-(S-2-methyl-)-butylester, 32 g (127 mmol) 11-Brom-1-undecanol und 20 g (145 mmol) wasserfreies Kaliumcarbonat wurden in 500 ml Dimethylformamid bei 100°C bis zur vollständigen Umsetzung des Hydroxybiphenylcarbonsäureesters gerührt, die dünnschichtchromtographisch festgestellt wurde. Nach dem Abkühlen auf Raumtemperatur wurde festes Nebenprodukt abgetrennt und verworfen. Aus dem Filtrat wurde das Lösungsmittel entfernt und das feste Rohprodukt zweimal aus Aceton umkristallisiert.
   Ausbeute: 45 %
b) 2-Hydroxy-1,2,3-propantricarbonsäuretri-(4-(4'-undecyloxy)-biphenylcarbonsäure-(S-2-methyl-)butylester-)yl-ester
   0,8 g (3,6 mmol) Citronensäure, 5 g (11 mmol) 4-(4'-ω-Hydroxyundecyloxy)-biphenylcarbonsäure-(S-2-methyl-)butylester und 1 g p-Toluolsulfonsäure wurden in 150 ml wasserfreiem Toluol unter Rückfluß erhitzt. Die Dampfphase wurde kondensiert, von darin enthaltenem Wasser befreit, und die Toluolphase wurde zurückgeführt. Nach vollständiger Umsetzung wurden Lösungsmittel und p-Toluolsulfonsäure entfernt und der Rückstand durch Chromatographie über Kieselgel (Elutionsmittel:Toluol/Essigester=5/1) und Umkristallisation aus Methanol gereinigt.
   Ausbeute: 30 %
c) Bis-1,2,3-propantricarbonsäuretri-(4-(4'-undecyloxy)-biphenylcarbonsäure-(S-2-methyl-)butylester)-yl-adipat
   3,5 g (2,3 mmol) 2-Hydroxy-1,2,3-propantricarbonsäuretri-(4-(4'-undecyloxy)-biphenylcarbonsäure-(S-2-methyl-)butylester)-yl-ester, 0,3 ml Triethylamin und 50 mg 4-Dimethylaminopyridin wurden in 100 ml Methylenchlorid bei einer Temperatur von -5 bis -10°C mit 0,27 ml (1,2 mmol) Adipinsäuredichlorid versetzt, 30 Minuten bei dieser Temperatur gerührt, auf Raumtemperatur erwärmt und bis zur vollständigen Umsetzung des Citronensäurederivates gerührt.
   Anschließend wurde die Reaktionsmischung mit Wasser versetzt, die organische Phase abgetrennt, mit wasserfreiem Natriumsulfat getrocknet und das Lösungsmittel entfernt. Der Rückstand wurde in Tetrahydrofuran gelöst und mit Petrolether ausgefällt.
   Ausbeute: 82 %
   ¹H-NMR (CDCl₃, TMS):
   - δ (ppm):: 0,95-1,95 (m,CH,CH₂,CH₃); 2,75-2,95 (m,CH₂);
   3,95-4,30 (m,OCH₂); 6,90-8,10 (m, Arom.-H)

### Beispiel 5

### Glycerin-tri-(ω-(4'-cyanbiphenyl-4-oxy)-heptylcarboxylat)

a) Glycerin-tri-(ω-bromheptylcarboxylat)
   Zu einer Lösung, bestehend aus 9 g (100 mmol) Glycerin, 26 g (330 mmol) Pyridin und 300 ml Methylenchlorid, wurden 80 g (330 mmol) 8-Bromoctansäurechlorid - gelöst in 80 ml Methylenchlorid - zugegeben, die dann unter Feuchtigkeitsausschluß bei Raumtemperatur 24 Stunden gerührt wurde. Nach der Aufarbeitung wurden 68 g Produkt erhalten.
   Ausbeute: 96 %
b) Glycerin-tri-(ω-(4'-cyanbiphenyl-4-oxy)-heptylcarboxylat)
   7,1 g (10 mmol) Glycerin-tri-(ω-bromheptylcarboxylat), 6,2 g (32 mmol) 4-(4'-Hydroxybiphenyl)-carbonitril, 4,4 (32 mmol) frisch gemahlenes und geglühtes Kaliumcarbonat sowie 0,5 g Kaliumjodid wurden in 70 ml 2-Butanon 48 Stunden unter Rückfluß erhitzt. Danach wurde die Lösung auf Wasser gegeben, die organische Phase mit Essigester extrahiert und aufgearbeitet. Es wurden 8 g Rohprodukt erhalten, die durch Chromatographie über Kieselgel (Elutionsmittel: Toluol/Essigester = 1/1) gereinigt wurden.
   Ausbeute: 17 %
   ¹H-NMR (CDCl₃, TMS):
   - δ (ppm):: 1,2-1,35 (m,18H,CH₂); 1,55-1,7 (m,6H,CH₂);
   1,7-1,9 (m,6H,CH₂); 2,33(t,6H,CH₂); 3,97 (t,6H,CH₂);
   4,1-4,25 (m,2H,CH₂); 4,25-4,4 (m,2H,CH₂); 5,3 (m,1H,CH);
   6,93 (d,6H,Arom.-H); 7,5 (d,6H,Arom.-H);
   7,57-7,8 (m,12H,Arom.-H)
   - Phasenübergänge:: g - n 13°C
   n - i 91°C

### Beispiel 6

### Glycerin-tri-(ω-(4'-cyanbiphenyl-4-oxy)-decylcarboxylat)

a) Glycerin-tri-(ω-bromdecylcarboxylat)
   Die Herstellung erfolgte gemäß Beispiel 6a). Anstelle von 8-Bromoctansäurechlorid wurde 11-Bromundecansäurechlorid eingesetzt.
b) Glycerin-tri-(ω-(4'-cyanbiphenyl-4-oxy)-decylcarboxylat)
   Die Herstellung erfolgte gemäß Beispiel 6b). Anstelle von Glycerintri-(ω-bromheptylcarboxylat) wurde Glycerin-tri-(ω-bromdecylcarboxylat) eingesetzt.
   Ausbeute: 15 %
   ¹H-NMR (CDCl₃, TMS):
   - δ (ppm):: 1,2-1,4 (m,30H,CH₂); 1,4-1,5 (m,6H,CH₂); 1,5-1,7 (m,6H,CH₂);
   1,7-1,9 (m,6H,CH₂); 2,3 (t,6H,CH₂); 4,0 (t,6H,CH₂);
   4,1-4,2 (m,2H,CH₂) ; 4,26-4,35 (m,2H,CH₂) ; 5, 28 (m,1H,CH);
   6,95 (d,6H,Arom.-H); 7,53 (d,6H,Arom.-H); 7,55-7,8 (m,12H,Arom.-H)
   - Phasenübergänge:: g - s 8°C
   s - n 84°C
   n - i 90°C

### Herstellung der flüssigkristallinen Verbindungen II

### Beispiel 7

### 3,4,5-Tri-(4-(4'-hexyloxy)-biphenylcarbonitril-oxy)-benzoesäuremethylester

1,8 g (10 mmol) 3,4,5-Trihydroxybenzoesäuremethylester, 10,7 g (30 mmol) 4-(4'-ω-Bromhexyloxy)-biphenylcarbonitril, 4,1 g (30 mmol) frisch gemahlenes und geglühtes Kaliumcarbonat, 5 g (30 mmol) Kaliumiodid und 50 ml wasserfreies Nitrobenzol wurden unter Rühren 8 Stunden auf 150°C erhitzt. Nach dem Abkühlen auf Raumtemperatur wurde die Suspension abfiltriert, Nitrobenzol aus dem Filtrat destilliert und der Rückstand durch Chromatographie über Kieselgel (Elutionsmittel: Toluol/Essigester = 2/1) gereinigt. Anschließend wurde die Hauptfraktion in 30 ml Methylenchlorid gelöst und unter Rühren in 200 ml kaltes Methanol getropft. Das ausgefallene Produkt wurde abfiltriert, mit kaltem Methanol gewaschen und getrocknet.
Ausbeute: 39 %
¹H-NMR (CDCl₃, TMS):
- δ (ppm):: 1,4-1,5 (m,12H,CH₂); 1,5-2,1 (m,12H,CH₂); 3,92 (s,3H,OCH₃);
3,8-4,2 (m,12H,CH₂-O); 6,8-7,1 (m,6H,Arom.-H); 7,28 (s,2H,Arom.-H); 7,3-7,8 (m,18H,Arom.-H)
- Phasenübergänge:: k - g 127°C
g - n (27°C)
n - i (125°C)

### Beispiel 8

### 1,3,5-Tri-(4-(4'-hexyloxy)-biphenylcarbonitril-oxy)-benzol

Die Herstellung erfolgte gemäß Beispiel 7., Anstelle von 3,4,5-Trihydroxybenzoesäuremethylester wurde 1,3,5-Trihydroxybenzol eingesetzt.
Ausbeute: 35 %
¹H-NMR (CDCl₃, TMS):
- δ (ppm):: 1,36-1,7 (m,12H,CH₂); 1,7-2,1 (m,12H,CH₂); 3,93 (t,6H,CH₂-O);
4,02 (t,6H,CH₂-O); 6,09 (s,3H,Arom.-H); 7,0 (d,6H,Arom.-H);
7,4-7,8(m,18H,Arom.-H)
- Phasenübergänge:: g - n 25°C
n - i 116°C

### Beispiel 9

### Phloroglucin-tri-(ω-(4'-cyanbiphenyl-4-oxy)-heptylcarboxylat)

Die Herstellung erfolgte gemäß Beispiel 1c). Anstelle von Pentaerythrit wurde 1,3,5-Trihydroxybenzol mit 8-(4'-Cyanbiphenyl-4-oxy)-octansäurechlorid umgesetzt.
Ausbeute: 35 %
¹H-NMR (DMSO-d₆, TMS):
- δ (ppm):: 1,1-1,55 (m,18H,CH₂); 1,55-1,9 (m,12H,CH₂); 2,52 (t,6H,CH₂);
4,0 (t,6H,CH₂); 6,9 (s,3H,Arom.-H); 7,03 (d,6H,Arom.-H);
7,66 (d,6H,Arom.-H); 7,72-8,0 (m,12H,Arom.-H)
- Phasenübergänge:: k - g 114°C
g - n 22°C
n - i 98°C

### Beispiel 10

### 1,2,4,5-Benzoltetracarbonsäure-tetra-(4-(4'-hexyloxy)-biphenylcarbonitril)-yl-ester)

1,1 g (5 mmol) Pyromellitsäureanhydrid, 5,2 g (20 mmol) 4-(4'-ω-Hydroxyhexyloxy)-biphenylcarbonitril, 0,2 g p-Toluolsulfonsäure und 50 ml Xylol wurden 12 Stunden unter Rückfluß am Wasserabscheider erhitzt. Nach dem Abkühlen wurden 100 ml Methylenchlorid zugegeben. Der nach der Aufarbeitung verbleibende Rückstand wurde durch Chromatographie über Kieselgel (Elutionsmittel: Toluol/Essigsäure = 4/1) und die Hauptfraktion durch Umkristallisation aus Toluol gereinigt.
Ausbeute: 32 %
¹H-NMR (DMSO-d₆, TMS):
- δ (ppm):: 1,3-1,52 (m,16H,CH₂); 1,6-1,8 (m,16H,CH₂); 3,97 (t,8H,CH₂) ;
4,3 (t,8H,CH₂); 7,0 (d,8H,Arom.-H); 7,65 (d,8H,Arom.-H);
7,72-7,9 (m,16H,Arom.-H); 8,1 (s,2H,Arom.-H);
- Phasenübergänge:: k - n 140°C
n - i (98°C)

### Beispiel 11

### Bestimmung der Schaltzeiten

Es wurden die schnellsten Schaltzeiten τ von drei flüssigkristallinen Verbindungen (Beispiele 6, 7 und 8) sowie eines polymeren Flüssigkristalls, das aus der Monomereinheit 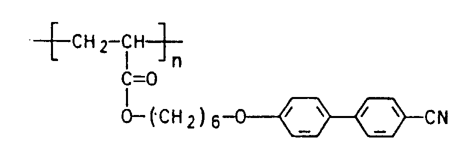 aufgebaut ist, bestimmt.

Für die Messungen wurden jeweils 2 µm-Zellen mit angeriebener Polyimidschicht der Firma EHC Co. Ltd. verwendet. Die Schaltversuche wurden alle mit einer Rechteckspannung von ±15 V und 1 kHz durchgeführt.

**Tabelle**

| Flüssigkristall | Klärpunkt (T/°C) | Schaltbereich (°C) | schnellste Schaltzeit (τ/ms) |
|---|---|---|---|
| Beispiel 7 | 90,0 | 78 - 89,5 | 1,98 (89,5°C) |
| Beispiel 8 | 125,7 | 65 - 125,5 | 0,70 (125,5°C) |
| Beispiel 9 | 117,5 | 65 - 116,0 | 2,24 (116°C) |
| Polymer | 124,5 | 116 - 124,0 | 20,20 (145°C) |

## Patentansprüche

1. Flüssigkristalline Verbindungen der allgemeinen Formeln I und II,
Z¹-(X¹-R¹-Y-M)ₘ I
Z²-(X²-R¹-Y-M)ₙ II
in denen die Variablen folgende Beutung haben:
Z¹ der Alkoholat- oder Säurerest Z¹/1 eines m-wertigen aliphatischen Alkohols bzw. einer m-wertigen aliphatischen Carbonsäure mit 3 bis 30 C-Atomen;
derAlkoholat-oder Säurerest Z¹/2 eines m-wertigen cycloaliphatischen Alkohols bzw. einer m-wertigen cycloaliphatischen Carbonsäure mit 5- oder 6-Ringgliedern;
für den Fall m = 3 der stickstoffhaltige Rest Z¹/3. 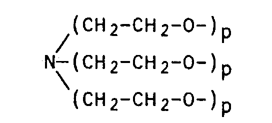 wobei p 1 oder 2 sein kann;
für den Fall m = 3 ein Rest der Strukturen Z¹/4a-d 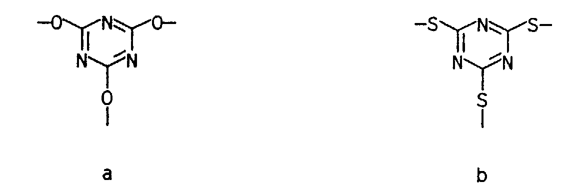 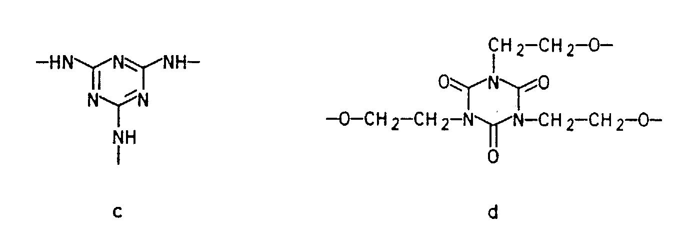 für den Fall m = 3 oder m = 4 der Säurerest Z¹/5 der Nitrilotriessigsäure bzw. der Ethylendiaminotetraessigsäure;
der Rest Z¹/6 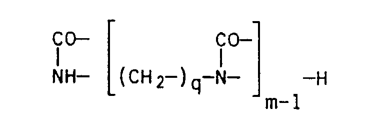 wobei q 2 oder 3 sein kann;
Z² der n-wertige Rest Z²/1 eines Benzols, 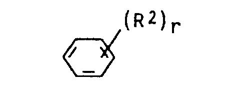 wobei R² Halogen, Cyano, Nitro, C₁- bis C₁₀-Alkyl, C₁- bis C₁₀-Alkoxy, C₁- bis C₁₀-Alkoxycarbonyl, C₁- bis C₁₀-Acyloxy oder Reste, die in vicinaler Stellung zu einem Ring verbunden sind, bedeuten kann, r für Null bis 3 steht und die Reste R² im Falle r > 1 gleich oder verschieden sein können;
der mehrkernige Rest Z²/2 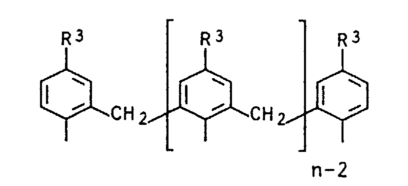 wobei R³ C₁- bis C₄-Alkyl, C₁- bis C₄-Alkoxy oder Halogen bedeuten kann;
für den Fall n = 3 der phosphorhaltige Rest Z²/3, 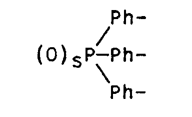 wobei s Null oder 1 sein kann;
X¹ eine chemische Bindung oder eine -CO- Gruppe;
X² Sauerstoff, Schwefel, -CO-O-, -O-CO-, -SO₂-, -SO₂-O-, -O-SO₂-O-, -NR⁴-, -CO-NR⁴-, -NR⁴-O- oder -CO-N〈,
wobei R⁴ Wasserstoff oder C₁- bis C₈-Alkyl bedeuten kann und die Maßgabe gilt, daß X² im Falle des mehrkernigen Restes Z²/2 nur -O- oder -O-CO- bedeutet;
m 3 bis 6, mit der Maßgabe, daß m gleich oder kleiner der Anzahl der C-Atome in Z¹ ist;
n 3 bis 6;
R¹ eine C₂- bis C₂₀-Brücke mit 2 bis 12 Brückengliedern, die durch Sauerstoff, Schwefel oder -NR⁴- unterbrochen sein können, und wobei diese Heteroeinheiten jeweils durch mindestens 2 C-Atome getrennt sind;
Y eine chemische Bindung, Sauerstoff, Schwefel, -CO-O-, -O-CO-, -NR⁴-, -CO-NR⁴- oder -NR⁴-CO-;
M eine mesogene Gruppe, die sich von einer Verbindung ableitet, welche in der flüssigkristallinen Phase eine Anisotropie der Dielektrizitätskonstanten ε mit |Δε| > 0,3 aufweist und/oder welche optisch aktiv ist und durch die allgemeinen Formeln III oder IV dargestellt wird:
-A-(B-A-)ₜ (D¹)ᵤ III
-A-(B-A-)ₜ (D²)ᵤ IV
in denen die Variablen die folgende Bedeutung haben:
A eine 1,4-Phenylen- oder 2,6-Naphthylengruppe, die bis zu zwei Stickstoffatome als Heteroatome enthalten kann, und die bis zu zwei der folgenden Substituenten tragen kann: Fluor, Chlor, Brom, Nitro oder Cyano;
eine 1,4-Cyclohexylengruppe, die bis zu zwei der folgenden Heteroatome in jeweils nicht benachbarter Stellung enthalten kann: Sauerstoff, Schwefel oder Stickstoff;
B eine chemische Bindung oder eines der folgenden Brückenglieder: -CO-O-, -O-CO-, -CH₂-CH₂-, -CH₂-O-, -O-CH₂-, -CH₂-S-, -S-CH₂, -C≡C-, -CH=CH-, -CH=N-, -N=CH-, -N=N-, 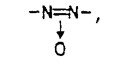 -CH=CH-CO-O- oder -O-CO-CH=CH-;
D¹ einer der folgenden Substituenten:
Fluor, Chlor, Brom, Nitro, Cyano, -OCFH₂, -OCF₂H, -OCF₃, -O-CO-R⁵ oder -CO-O-R⁵
wobei R⁵ ein linearer C₁- bis C₂₀-Alkylrest, der durch Sauerstoff unterbrochen sein kann, und der durch Fluor, Chlor, Brom, Cyano oder Methyl asymmetrisch substituiert sein kann, bedeutet;
t 1 bis 4, mit der Maßgabe, daß die Gruppen A und B verschieden sein können;
u 1, wenn D¹ bzw. D² mit einem aromatischen Ring verknüpft ist, und 1 oder 2, wenn D¹ bzw. D² mit einem Cyclohexylenring verknüpft ist,
D² eine der folgenden enantiomeren Gruppen:
eine lineare C₁- bis C₂₀-Alkylgruppe, die an ein oder zwei Kohlenstoffatomen durch Fluor, Chlor, Brom, Cyano, Trifluormethyl oder Methyl asymmetrisch substituiert ist, und die einmal durch die Gruppierung -CO-O- oder bis zu zweimal durch die Gruppierung -O- unterbrochen sein kann, oder
eine lineare C₃- bis C₆-Alkylgruppe, die durch die Gruppierung 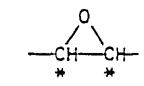 unterbrochen ist,
mit der Maßgabe, daß diese enantiomeren Gruppen D² auch durch -O-, -CO-O- oder -O-CO- mit A verknüpft sein können,
ausgenommen die Verbindung Tetrakis(5-(4'-cyano-biphenylyl-4)-valeryloxymethyl)methan entsprechend einer Verbindung der Formel I.

2. Verfahren zur Herstellung der flüssigkristallinen Verbindungen I gemäß Anspruch 1, dadurch gekennzeichnet, daß man die Verbindungen Z¹(H)ₘ, Z¹(Cl)ₘ oder (Z¹)₂O in an sich bekannter Weise mit einem Carbonsäurehalogenid M-Y-R¹-CO-Hal bzw. einem Alkohol M-Y-R¹-O-H umsetzt, wobei Hal für Chlor oder Brom steht.

3. Verfahren zur Herstellung der flüssigkristallinen Verbindungen I gemäß Anspruch 1, dadurch gekennzeichnet, daß man die Verbindung Z¹(H)ₘ in an sich bekannter Weise mit einem ω-Bromcarbonsäurechlorid Br-R¹-CO-Cl umsetzt und das Zwischenprodukt Z¹-CO-R¹-Br mit der Verbindung HO-M verethert.

4. Verfahren zur Herstellung der flüssigkristallinen Verbindungen II gemäß Anspruch 1, dadurch gekennzeichnet, daß man die Verbindung Z²(OH)ₙ in an sich bekannter Weise mit Verbindungen, die sich von der Verbindung M-Y-R¹-X²-H ableiten, bzw. einem Alkylbromid M-Y-R¹-Br umsetzt.

5. Verfahren zur Herstellung der flüssigkristallinen Verbindungen II gemäß Anspruch 1, dadurch gekennzeichnet, daß man die Verbindung Z²(OH)ₙ in an sich bekannter Weise mit ω-Bromcarbonsäurechlorid Br-R¹-CO-Cl umsetzt und das Zwischenprodukt Z²-O-CO-R¹-Br mit der Verbindung HO-M verethert.

6. Verfahren zur Herstellung der flüssigkristallinen Verbindungen II gemäß Anspruch 1, dadurch gekennzeichnet, daß man die Verbindungen Z²(COOH)ₙ, Z²(COCl)ₙ und (Z₂-CO)₂O in an sich bekannter Weise mit einer Verbindung M-Y-R¹-OH umsetzt.

7. Verwendung der flüssigkristallinen Verbindungen I und II gemäß Anspruch 1 allein, in Mischungen untereinander und mit anderen flüssigkristallinen und/oder nicht flüssigkristallinen Verbindungen in Form fester oder flüssigkristalliner optisch anisotroper Medien für die Darstellung und Speicherung von Informationen.

## Claims

1. A liquid-crystalline compound of the formula I or II
Z¹-(X¹-R¹-Y-M)ₘ I
Z²- (X²-R¹-Y-M)ₙ II
where
Z¹ is the alcoholate or acid radical Z¹/1 of an m-valent aliphatic alcohol or of an m-valent aliphatic carboxylic acid having 3 to 30 carbon atoms,
the alcoholate or acid radical Z¹/2 of an m-valent cycloaliphatic alcohol or of an m-valent cycloaliphatic carboxylic acid having 5 or 6 ring members,
in the case where m = 3, the nitrogen-containing radical Z¹/3 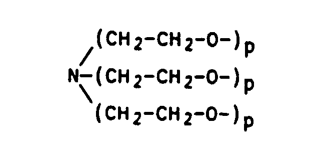 where p may be 1 or 2,
in the case where m = 3, a radical having the structure Z¹/4a-d 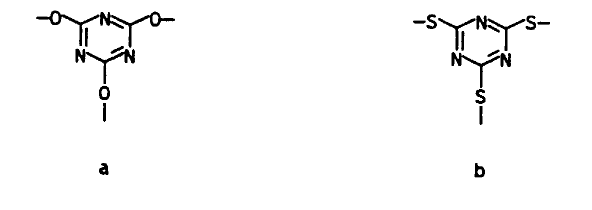 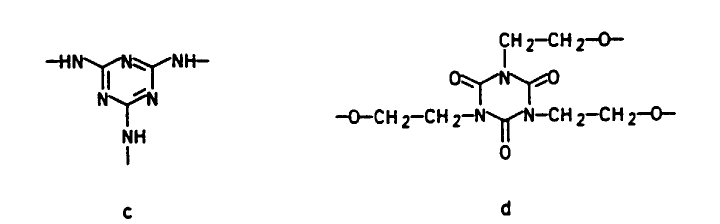 in the case where m = 3 or m = 4, the acid radical Z¹/5 of nitrilotriacetic acid or of ethylenediaminetetraacetic acid,
the radical Z¹/6 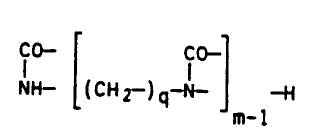 where q may be 2 or 3,
Z² is the n-valent radical Z²/1 of a benzene 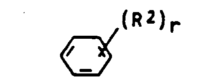 where R² may be halogen, cyano, nitro, C₁-C₁₀-alkyl, C₁- to C₁₀-alkoxy, C₁- to C₁₀-alkoxycarbonyl, C₁- to C₁₀-acyloxy or radicals which are bonded to a ring in the vicinal position, r is zero to 3, and the radicals R², in the case where r > 1, may be identical or different,
the polycyclic radical Z²/2 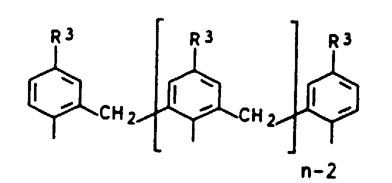 where R³ may be C₁- to C₄-alkyl, C₁- to C₄-alkoxy or halogen,
in the case where n = 3, the phosphorus-containing radical Z²/3 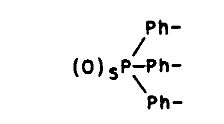 where s may be zero or 1,
X¹ is a chemical bond or -CO-,
X² is oxygen, sulfur, -CO-O-, -O-CO-, -SO₂-, -SO₂-O-, -O-SO₂-O-, -NR⁴-, -CO-NR⁴-, -NR⁴-O- or -CO-N<,
where R⁴ may be hydrogen or C₁- to C₈-alkyl, with the proviso that, in the case of the polycyclic radical Z²/2, X² can only be -O- or -O-CO-,
m is 3 to 6, with the proviso that m is less than or equal to the number of carbon atoms in Z¹,
n is 3 to 6,
R¹ is a C₂- to C₂₀-bridge containing 2 to 12 bridging members, which may be interrupted by oxygen, sulfur or -NR⁴-, it being possible for each of these hetero units to be separated by at least 2 carbon atoms,
Y is a chemical bond, oxygen, sulfur, -CO-O-, -O-CO-, -NR⁴-, -CO-NR⁴- or -NR⁴-CO-, and
M is a mesogenic group derived from a compound which, in the liquid-crystalline phase, has an anisotropy of the dielectric constant ε where |Δε| > 0.3 and/or which is optically active and is represented by the formula III or IV:
-A-(B-A-)ₜ (D¹)ᵤ III
-A-(B-A-)ₜ (D²)ᵤ IV
where
A is 1,4-phenylene or 2,6-naphthylene, which may contain up to two nitrogen atoms as hetero atoms and may carry up to two fluorine, chlorine, bromine, nitro or cyano substituents, or is 1,4-cyclohexylene, which may contain up to two hetero atoms from oxygen, sulfur and nitrogen, in each case in non-adjacent positions,
B is a chemical bond or one of the following bridging members:
-CO-O-, -O-CO-, -CH₂-CH₂-, -CH₂-O-, -O-CH₂-, -CH₂-S-, -S-CH₂-, -C≡C-, -CH=CH-, -CH=N-, -N=CH-, -N=N-, 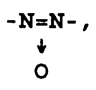 -CH=CH-CO-O- and -O-CO-CH=CH-,
D¹ is a fluorine, chlorine, bromine, nitro, cyano, -OCFH₂, -OCF₂H, -OCF₃, -O-CO-R⁵ or -CO-O-R⁵ substituent, where R⁵ is linear C₁- to C₂₀-alkyl, which may be interrupted by oxygen and may be asymmetrically substituted by fluorine, chlorine, bromine, cyano or methyl,
t is a number from 1 to 4, with the proviso that A and B may be different from one another, and
u is 1 if D¹ or D² is linked to an aromatic ring and 1 or 2 if D¹ or D² is linked to a cyclohexylene ring,
D² is one of the following enantiomeric groups:
linear C₁- to C₂₀-alkyl which is asymmetrically substituted on one or two carbon atoms by fluorine, chlorine, bromine, cyano, trifluoromethyl or methyl and may be interrupted once by -CO-O- or up to twice by -O-,
or
linear C₃- to C₆-alkyl which is interrupted by 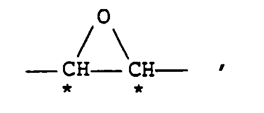 with the proviso that these enantiomeric groups D² may be linked to A via -O-, -CO-O- or -O-CO-,
with the exception of the compound tetrakis(5-(4'-cyanobiphenyl-4-yl)valeryloxymethyl)methane corresponding to a compound of the formula I.

2. A process for the preparation of a liquid-crystalline compound I as claimed in claim 1, which comprises reacting a compound Z¹(H)ₘ, Z¹(Cl)ₘ or (Z¹)₂O in a manner known per se with a carboxylic acid halide M-Y-R¹-CO-Hal or with an alcohol M-Y-R¹-O-H, where Hal is chlorine or bromine.

3. A process for the preparation of a liquid-crystalline compound I as claimed in claim 1, which comprises reacting a compound Z¹(H)ₘ in a manner known per se with an ω-bromocarboxylic acid chloride Br-R¹-CO-Cl, and etherifying the intermediate Z¹-CO-R¹-Br using a compound HO-M.

4. A process for the preparation of a liquid-crystalline compound II as claimed in claim 1, which comprises reacting a compound Z²(OH)ₙ in a manner known per se with a compound derived from the compound M-Y-R¹-X²-H or with an alkyl bromide M-Y-R¹-Br.

5. A process for the preparation of a liquid-crystalline compound II as claimed in claim 1, which comprises reacting a compound Z²(OH)ₙ in a manner known per se with an ω-bromocarboxylic acid chloride Br-R¹-CO-Cl, and etherifying the intermediate Z²-O-CO-R¹-Br using a compound HO-M.

6. A process for the preparation of a liquid-crystalline compound II as claimed in claim 1, which comprises reacting a compound Z²(COOH)ₙ, Z²(COCl)ₙ or (Z²-CO)₂O in a manner known per se with a compound M-Y-R¹-OH.

7. A method of using a liquid-crystalline compound I or II as claimed in claim 1, alone, in a mixture with one another or with other liquid-crystalline and/or non-liquid-crystalline compounds, in the form of solid or liquid-crystalline, optically anisotropic media for the display and storage of information.

## Revendications

1. Composés cristaux liquides de formules générales I et II,
Z¹-(X¹-R¹-Y-M)ₘ I
Z²-(X²-R¹-Y-M)ₙ II
dans lequelles les variables ont les significations suivantes :
Z¹ le reste alcoolate ou le reste acide Z¹/1 d'un alcool aliphatique ayant m valences, respectivement d'un acide carboxylique aliphatique ayant m valences et 3 à 30 atomes de carbone ;
le reste alcoolate ou le reste acide Z¹/2 d'un alcool cycloaliphatique ayant m valences, respectivement d'un acide carboxylique cycloaliphatique à 5 ou 6 maillons dans le cycle et ayant m valences ;
dans le cas où m = 3, le reste azoté Z¹/3, 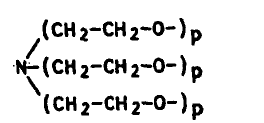 où p peut valoir 1 ou 2 ;
dans le cas où m = 3, un reste de structure Z¹/4a-d 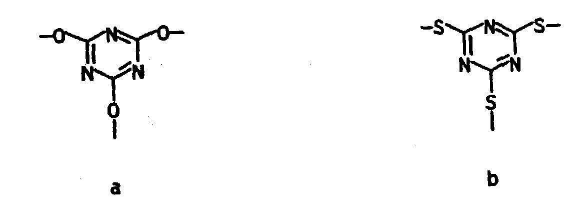 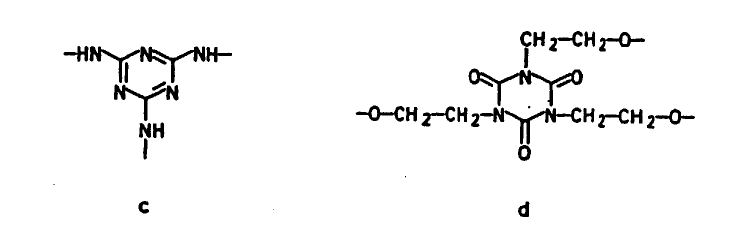 dans le cas où m = 3 ou m = 4, le reste acide Z¹/5 de l'acide nitrilotriacétique, respectivement de l'acide éthylènediamine-tétracétique ;
le reste Z¹/6 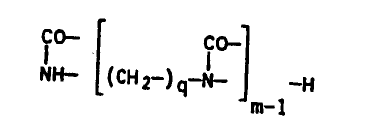 où q peut valoir 2 ou 3 ;
Z² le reste Z²/1 à n valences d'un composé benzénique 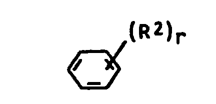 où R² peut représenter un atome d'halogène, des groupements cyano, nitro, alkyle en C₁-C₁₀, alcoxy en C₁-C₁₀, (alcoxy en C₁-C₁₀)carbonyle, acyloxy en C₁-C₁₀ ou bien des restes reliés en position vicinale par rapport à un cycle, r vaut de 0 à 3 et les restes R² peuvent être identiques ou différents lorsque r > 1 ;
le reste Z²/2 à plusieurs cycles 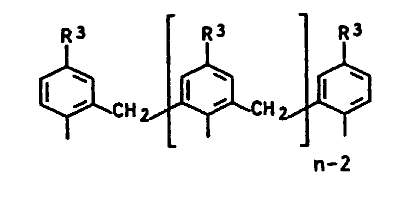 où R³ peut représenter des groupements alkyle en C₁-C₄, alcoxy en C₁-C₄ ou un atome d'halogène ;
dans le cas où n = 3, le reste phosphoré Z²/3, 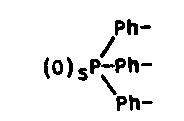 où s peut valoir 0 ou 1 ;
X¹ une liaison chimique ou un groupement -CO-;
X² un atome d'oxygène, un atome de soufre, -CO-O-, -O-CO-, -SO₂-, -SO₂-O-, -O-SO₂-O-, -NR⁴-, -CO-NR⁴-, -NR⁴-O- ou -CO-N<,
où R⁴ peut représenter un atome d'hydrogène ou un groupement alkyle en C₁-C₈, étant spécifié que X² ne peut représenter que -O- ou -O-CO- dans l'hypothèse du reste Z²/2 à plusieurs cycles ;
m 3 à 6, étant spécifié que m est inférieur ou égal au nombre d'atomes de carbone dans Z¹ ;
n 3 à 6;
R¹ un pont en C₂-C₂₀ avec 2 à 12 maillons pontants, pouvant être interrompus par des atomes d'oxygène, de soufre ou des groupements -NR⁴-, et où chacun de ces motifs à hétéroatomes est séparé par au moins 2 atomes de carbone ;
Y une liaison chimique, un atome d'oxygène, de soufre, -CO-O-, -O-CO-, -NR⁴-, -CO-NR⁴ ou NR⁴-CO- ;
M un groupement mésogène dérivant d'un composé qui présente en phase liquide cristalline une anisotropie de sa constante diélectrique ε telle que |Δε| > 0,3 et/ou qui est optiquement actif et peut être représenté par les formules générales III ou IV
-A-(B-A-)ₜ (D¹)ᵤ III
-A-(B-A-)ₜ (D²)ᵤ IV
dans lequelles les variables ont les significations suivantes :
A un groupement 1,4-phénylène ou 2,6-napthylène pouvant contenir jusqu'à deux atomes d'azote en tant qu'hétéroatomes et pouvant porter jusqu'à deux des substituants suivants : atomes de fluor, chlore, brome, groupements nitro ou cyano ;
un groupement 1,4-cyclohexylène pouvant contenir jusqu'à deux des hétéroatomes suivants, en position non voisine à chaque fois : oxygène, soufre ou azote ;
B une liaison chimique ou l'un des maillons pontants suivants :
-CO-O-, -O-CO-, -CH₂-CH₂-, -CH₂-O-, -O-CH₂-, -CH₂-S-, -S-CH₂, -C≡C-, -CH=CH-, -CH=N-, -N=CH-, -N=N-, 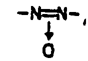 -CH=CH-CO-O- ou -O-CO-CH=CH-;
D¹ un des substituants suivants :
atomes de fluor, de chlore, de brome, groupements nitro, cyano, -OCHFH₂, -OCF₂H, -OCF₃, -O-CO-R⁵ ou -CO-O-R⁵,
où R⁵ représente un reste alkyle linéaire en C₁-C₂₀ pouvant être interrompu par des atomes d'oxygène et pouvant être substitué de façon asymétrique par des atomes de fluor, de chlore, de brome, par des groupements cyano ou méthyle ;
t 1 à 4, étant spécifié que les groupements A et B peuvent être différents ;
u 1, lorsque D¹, respectivement D², est lié à un cycle aromatique, et 1 ou 2 lorsque D¹, respectivement D², est lié à un cycle cyclohexylène,
D² un des groupements énantiomères suivants :
un groupement alkyle en C₁-C₂₀ linéaire substitué de façon asymétrique, sur un ou deux atomes de carbone, par des atomes de fluor, de chlore, de brome, par des groupements cyano, trifluorométhyle ou méthyle, et pouvant être interrompu une fois par le groupement -CO-O- ou jusqu'à deux fois par le groupement -O-, ou
un groupement alkyle en C₃-C₆ linéaire interrompu par le groupement 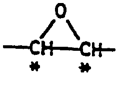 étant spécifié que ces groupements énantiomères D² peuvent aussi être reliés à A par -O-, -CO-O- ou -O-CO-,
à l'exception du composé tétrakis(5-(4'-cyano-biphénylyl-4)valéryloxyméthyl)méthane correspondant à un composé de formule I.

2. Procédé de préparation des composés I cristaux liquides selon la revendication 1, caractérisé en ce que l'on fait réagir de façon connue en soi les composés Z¹(H)ₘ, Z¹(Cl)ₘ ou (Z¹)₂O avec un halogénure d'acyle M-Y-R¹-CO-Hal ou avec un alcool M-Y-R¹-OH, Hal étant mis pour un atome de chlore ou de brome.

3. Procédé de préparation des composés I cristaux liquides selon la revendication 1, caractérisé en ce que l'on fait réagir de façon connue en soi le composé Z¹(H)ₘ avec un chlorure d'acyle ω-bromé Br-R¹-CO-Cl et l'on éthérifie le produit intermédiaire Z¹-CO-R¹-Br avec le composé HO-M.

4. Procédé de préparation des composés II cristaux liquides selon la revendication 1, caractérisé en ce que l'on fait réagir le composé Z²(OH)ₙ de façon connue en soi avec des composés dérivant du composé M-Y-R¹-X²-H ou avec un bromure d'alkyle M-Y-R¹-Br.

5. Procédé de préparation des composés II cristaux liquides selon la revendication 1, caractérisé en ce que l'on fait réagir le composé Z²(OH)ₙ de façon connue en soi avec un chlorure d'acyle ω-bromé Br-R¹-CO-Cl et l'on éthérifie le produit intermédiaire Z²-O-CO-R¹-Br avec le composé HO-M.

6. Procédé de préparation des composés II cristaux liquides selon la revendication 1, caractérisé en ce que l'on fait réagir les composés Z²(COOH)ₙ, Z²(COCl)ₙ et (Z²-CO)₂O de façon connue en soi avec le composé M-Y-R¹-OH.

7. Utilisation des composés cristaux liquides I et II selon la revendication 1, seuls, en mélange entre eux et avec d'autres composés cristaux liquides et/ou non cristaux liquides sous forme de milieux optiquement anisotropes solides ou liquides cristallins pour l'affichage et l'enregistrement d'informations.
